# EUROPEAN PATENT SPECIFICATION

(11) **EP 2 887 980 B1**
(45) Date of publication and mention of the grant of the patent: **03.02.2016**
(21) Application number: 14702456.6
(22) Date of filing: 03.02.2014
(51) Int. Cl.: A61M 5/31, A61L 31/08, B05D 1/00, B05D 7/00, B65D 83/00, C08J 7/04, C23C 16/00, A61M 39/08, A61B 5/15

(54) **HYDROPHILICALLY INTERNALLY COATED MEDICAL DEVICE**
INTERN HYDROPHIL BESCHICHTETES MEDIZINPRODUKT
DISPOSITIF MÉDICAL REVÊTU INTÉRIEUREMENT D'UNE ENDUCTION HYDROPHILE

(30) Priority: 15.04.2013 EP 13001970
(43) Date of publication of application: 01.07.2015
(73) Proprietor: ABAG Aktienmarkt Beteiligungs AG, 50668 Köln (DE)
(72) Inventor: GÖRNE, Martin, 22337 Hamburg (DE)
(74) Representative: WSL Patentanwälte Partnerschaft mbB
(86) International application number: PCT/EP2014/000277
(87) International publication number: WO 2014/169977

(56) References cited:
- US-A1- 2003 031 806
- US-A1- 2010 132 762
- US-A1- 2012 123 345

## Description

The present invention relates to an inwardly hydrophilically coated medicinal-technical device for temporally accommodating therein a bodily liquid, in particular blood, or a cell suspension. In particular, the device may be a bottle, a bag, a tube, or a pipe, but may also be an actuatable part of an apparatus for applying an external action, such as pressure, heat or radiation, on the bodily liquid or blood or cell suspension, respectively.

Devices for temporally accommodating blood are known, some of which are for flowing through and some of which are for temporal storage. As an example, the blood tube disclosed in the German laid-open patent application DE 10 2011 108 787 A1 may be mentioned, the content of which is herein disclosed by reference insofar relating to the die arrangement of a blood guiding system.

A general phenomenon of such devices is the increase of the tendency of the blood, or certain protein components in the bodily liquid, to coagulate due to the contact with the device. This effect in most applications is undesirable, and is counteracted, if necessary, by the administration of agents reducing the coagulation tendency. On the other hand, such agents have, as such, undesirable side effects; be it that blood preservations are used during a surgical operation, and the surgeon desires speedy stoppage of bleeding; or be it that a bodily liquid is desired to be returned or further processed as purely and uncontaminated as possible. Similar issues are encountered with cell suspensions intended for being administered to a human being or a vertebrate animal (or mammal).

It is known from the patent application US 2012/0123345 A1 to coat inner surfaces of vessels by inserting an electrode in den hollow space within the vessel, and igniting a plasma in the presence of a reactive gas. The (hollow) electrode simultaneously serves as a gas supply, while the (concave) counter electrode is arranged surrounding the vessel. With respect to the arrangement of the apparatus components, this patent application is likewise incorporated herein by reference. The express purpose of this known method is to provide water-repellent surfaces. The inwardly coated vessels obtained in this manner are, however, in practice not yet satisfactory for temporary accommodation of bodily liquids, blood, or cell suspensions, respectively.

The present invention aims at diminishing, reducing or avoiding the disadvantages of the known devices and methods. To this end, the invention, under a first aspect, provides an inwardly coated hollow body for temporarily accommodating a bodily liquid, in particular blood, or cell suspensions, the hollow body having a hydrophilizing PECVD/CVD-coating on its inner surface.

Under a further aspect, the invention provides a process for coating a hollow body at its inner surface, the method comprising:
- pre-coating the inner surface using a reactive gas plasma (PECVD); and
- follow-up-coating the pre-coated surface with a reactive gas component without plasma action (CVD).

Under a still further aspect, the invention provides a use of acrylic acid or acrylic acid anhydride for hydrophilizing surfaces of a body, the use comprising:
- pre-coating the surfaces using a high frequency plasma generated from a gas mixture composed of an inert gas and a first gas formed of biocompatible, polymerizable, carboxy group-containing monomers, and then
- follow-up coating the pre-coated surfaces of the body,
wherein the pre-coating takes less than 10 minutes or even less than 1 minute, and the follow-up coating is made using a second gas substantially consisting of the acrylic acid or acrylic acid anhydride monomers.

Under the first aspect; a hollow body according to the invention has, at its inner surface, a hydrophilizing coating. Thereby, the tendency of the bodily liquid, blood, or cell suspension flown therethrough or accommodated therein is increased less than hitherto possible. In particular, a water contact angle, which designates the degree of hydrophily of a surface, may be set to less than 2° or even less than 0.5°. In embodiments, the surface material of the hollow body is a synthetic resin, and the hydrophilizing layer is made up of acrylic acid units. The hollow body may have exactly one opening, and may be a bottle or a bag; but it may also have two openings, through which, in use, the blood, bodily liquid, or cell suspension, respectively, is flown, and may be a pipe or a tube; or it may be an actuatable part of an apparatus applying, in operation, some external action, such as pressure, heat, or radiation, on the bodily liquid, blood, or cell suspension.

Under the second aspect, a method according to the invention may include an activating step, and includes a pre-treating step and a follow-up-treating step. Herein, in the activating step (if included), substantially only inert gas is present; in the pre-treating step, both inert gas as also a reactive gas is present; and in the terminal follow-up-treating step, substantially only the reactive gas is present, and there is no plasma action. In embodiments, the pre-treating takes less than 10 minutes, or less than 1 minute; the surface material of the hollow body may be made of a synthetic resin; and the coating material may be acrylic acid or its anhydride.

Without wishing to be bound by theory, it is assumed that the particularly short duration of the pre-treating step according to present invention allows for activated sites on the surface not to be immediately de-activated by the plasma, so that the two-step PECVD/CVD-coating particularly efficiently results in a hydrophilic surface.

Further advantages and features of the present invention will become apparent from the following detailed description of embodiments in conjunction with the drawings. The invention is not limited to the described embodiments, but by the scope of the appended claims. In particular, individual feature may be realized in embodiments according to the invention in a different number or und combination as in the examples described below. In the subsequent explanation of embodiments of the invention, reference is made to the accompanying drawings, which show:
- Figure 1: a schematic cross-section through a hollow body according to the invention, and
- Figure 2: a schematic flow diagram of a process according to the invention.

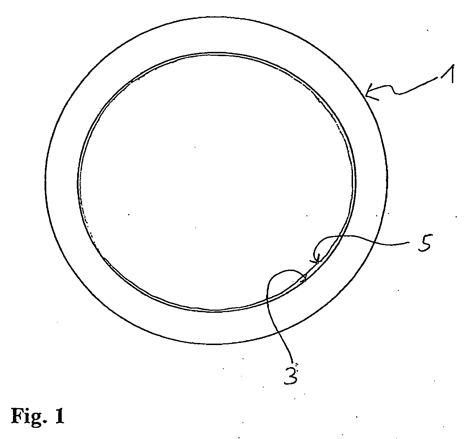

Figure 1 depicts a generally oval, in the embodiment shown: substantially circular cross section of the hollow body 1. Although the wall is shown as being unitary and seamless, it may in actual fact be multi-layered and/or may have one or more seams. The inner surface layer 3 is formed of a synthetic resin, which term shall designate cross-linked or non-cross-linked polymeric materials, the latter in particular in conjunction with two-(or more)layer walls, in which the mechanical stability is provided mainly by the outer layer or layers. The water contact angle of the inner surface layer is 10° or more, in particular 30° or more, and the surface layer will be termed "*little hydrophilic*" in this context. Through the combined PECVD/CVD-coating process with ultra-short pre-coating, described in detail below, a very thin adhesion layer is formed on the inner surface and on top of that, a thicker function layer 5, both made of carboxy group-containing units, e. g. acrylic acid units. These layers are stably bound to the inner surface layer and are surprisingly extremely hydrophilic, notably more hydrophilic than those obtained in an otherwise similar manner, but with a longer (> 1 minute, e.g. 10 minutes or more) pre-coating phase. It is thought that a longer pre-coating lets active sites, once generated, react further or with one another, in a manner detrimental to the hydrophilicity so obtainable. At the same time, a certain action time of the plasma is required for achieving a stable coating, such as at least 2 seconds.

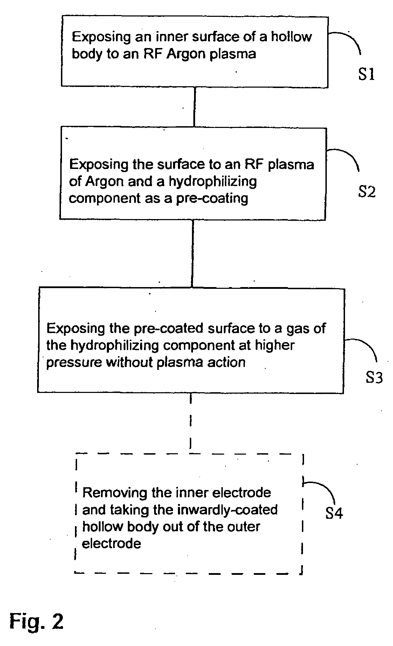

The flow diagram scheme of Figure 2 illustrates the important steps of a process for hydrophilizing inner surfaces by coating with poly(acrylic acid): After positioning a hollow electrode in the vessel or one-sidedly closed pipe or tube, same is evacuated by means of pumps, preferably to a pressure of maximally 10⁻⁴ mbar (10 mPa). After reaching the desired vacuum pressure, the interior of the vessel is purged with an inert gas, preferably Argon, while continuously pumping, wherein the inert gas supply is adjusted to the pumping speed so that a constant gas pressure is maintained in the interior of the vessel. The inert gas is supplied from an inert gas reservoir. In preferred embodiments, the Argon pressure so adjusted is about 25-150 mTorr (3.3-20 Pa). After reaching a stable inert gas pressure in the interior of the vessel, the plasma generator, for example a high frequency generator, is switched on for generating an inert gas plasma. The plasma cleans the inner surfaces by removing substances adsorbed thereto, and furthermore results in an activation of the inner surfaces by the formation of ions and free radicals, which enhance the subsequent graft polymerizing step. In some other embodiments, this pre-treatment step can be dispensed with.

The cleaning and activating effect in this first step S1 may be influenced *via* the frequency of the gas plasmas, the power fed into the plasma, the exposition time to the plasma and the kind of the inert gas used for the plasma. As the inert gas, in the process proposed here Argon is preferred, because it allows for an activation of the inner surfaces without generating new, undesired compounds. Naturally, instead other inert gases such as N₂ may be used. In an exemplary embodiment of the process, the exposition time to the Argon plasma is 20 to 120 seconds. After the exposition, the process is continued with the first coating step S2. The plasma generator may be switched off temporarily if desired.

Deviating from the above, the plasma may be generated on the basis of a mixture of the inert gas and a reactive component used in a subsequent pre-coating step, instead of on the basis of pure Argon. The partial pressure of the reactive component in the gas mixture should in this case be less than one tenth of the partial pressure of the inert gas.

On transitioning from step S1 to step S2 of the process, the inert gas supply into the interior of the vessel is preferably maintained and desirably adjusted so that it assumes a value suitable for carrying out step S2. For generating the gas mixture, a first coating material gas formed of carboxy group-containing monomers in the gas phase is admixed to the inert gas. The carboxy group-containing monomers may preferably be acrylic acid or a precursor of acrylic acid, such as e.g. acrylic acid anhydride. The partial pressure p_{eSG} of the first coating material gas in embodiments is at least one fourth of and maximally twice the partial pressure p_{IG} of the inert gas. It is particularly preferred that the partial pressure ratio p_{eSG}:p_{IG} is selected from a range of 1:1 to 1:0.5. For example, the partial pressure of Argon in embodiments of the process is 30 mTorr (ca. 400 mPa) at a total pressure of the gas mixture of 45 mTorr (ca. 600 mPa), resulting in a value of 2:1 for the ratio of the Argon partial pressure p_{Ar} to the partial pressure of the first coating material gas (reactive component partial pressure) p_{eSG}. While adjusting the desired reactive gas mixture the plasma generator may temporarily be switched off.

As the reactive component for forming the first coating material gas, preferably (meth)acrylic acid anhydride is used, which is evaporated in a separate container and is then guided to the interior of the vessel. The partial pressure of the coating material gas is adjusted via its supply, controlled by means of valves. Naturally, instead of (meth) acrylic acid anhydride (meth)acrylic acid may be used. (Meth)acrylic acid or (meth) acrylic acid anhydride, respectively, are provided in reservoir containers in liquid form, for example in an amount of 150 mL. In order to avoid or inhibit polymerisation of the acrylic acid or its precursor materials, respectively, same may be doted with Cu(I)-chloride. Furthermore, the reactive component containers are de-aerated after filling until no bubbles form in the reactive component liquid any longer. The vapour pressure of the reactive components usually is sufficient at normal room temperatures of 22 to 25°C for generating the first coating material gas.

After the desired gas mixture and gas mixture pressure have been formed, which typically takes 1 to 2 minutes, the actual coating process is initiated by starting the plasma generator, whereby excited acrylic acid monomers generated in the plasma attach to the activated inner surface and, in the subsequent process, form a poly(acrylic acid) layer. This plasma-enhanced pre-coating phase (PECVD) is maintained only for a short time in order to form a very thin adhesion layer. The pre-coating phase may take less than 60, or between 2 and 20 seconds, resulting in a particularly low contact angle for water such as below 2° or even below 0.5°. The gas supplies are preferably not varied during the plasma coating. In a first variant of the process, the pre-coating process is terminated by switching off the plasma generator.

Subsequent to the described first variant of the pre-coating step S2, a first variant of the follow-up-coating step S3 follows, in which after switching off the plasma generator initially the inert gas supply is interrupted and the pre-coated inner surface is exposed to, if possible, the full vapour pressure of the reactive component formed of water-free acrylic acid. This vapour pressure should not be below ca. 5 Torr (670 Pa). Slightly cooling or warming the reactive component in the reservoir container may be suitable for adjusting the pressure. Supplying the reactive component into the interior of the vessel at full vapour pressure provides reactive gas in large amounts, which gas reacts with the reactive centres present at the pre-coated surface and forms a relatively thick poly(acrylic acid) layer (PAA-layer) which may be crystalline.

In a second variant of the process, the plasma generator is not switched off at the end of the pre-coating step S2 and is therefore still in operation at the transition to the second variant of the follow-up-coating step S3. In this variant, the Argon supply is stopped almost or entirely, and the supply of the reactive gas, e. g. of the acrylic acid, is reduced to such an extent that, while maintaining high-frequency generation and continuously evacuating the interior of the vessel, a pressure equilibrium in the range of less than 0.3 mTorr (ca. 40 mPa) is adjusted. In an exemplary embodiment, the pressure is set to a pressure of less than 0.1 mTorr (ca. 13 mPa). Thereby, the plasma extinguishes due to the reduction in pressure. Otherwise, the second variant is similar to the first variant.

In both variants, the follow-up-coating phase is maintained for 1 to 45, or for 5 to 15 minutes.

After termination of the process in step S4, the inwardly coated vessels can be taken out of the apparatus along with the hollow electrode, and the inward coating may be inspected for quality evaluation, if desired.

The process described above allows for a long-term-stable hydrophilization of polymeric inner surface of hollow bodies or vessels, respectively, having a low tendency of coagulation of the bodily liquids or cell suspensions flown therethrough, or temporarily stored therein, or acted upon externally while in the hollow body.

The skilled person will realize that numerous modifications and augmentations to the above described embodiments are possible without leaving the scope of protection of the appended claims.

For example, while the above description relates to inner surfaces of hollow bodies, the process may likewise be applied to bodies of any shape, such as convex bodies, parts or components e.g. intended for use inside a hollow body. As another example, while the above description advocates an initial pre-treatment step, such step may be dispensed with if the surfaces are already sufficiently clean. In such a case, the initial step is the pre-coating step, and there is no preceding step in which the amount of reactive gas or its partial pressure is less than one tenth of the inert gas (or, respectively, its partial pressure), or in which the reactive gas is even absent.

## Claims

1. A hollow body for temporarily accommodating a bodily liquid, in particular blood, or cell suspensions, the hollow body having a hydrophilizing two-step PECVD/CVD-coating on its inner surface obtainable by the process of one of claims 8 to 11.

2. The hollow body according to claim 1, wherein a water contact angle of the coated inner surface is below 2°, in particular is below 0.5°.

3. The hollow body according to one of claims 1 to 2, having a polymeric coating made of acrylic acid monomers.

4. The hollow body according to one of claims 1 to 3, wherein a surface material of the hollow body is a synthetic resin.

5. The hollow body according to one of claims 1 to 4, having exactly one opening, the hollow body in particular being a bag or a bottle.

6. The hollow body according to one of claims 1 to 4, having two openings, between which the bodily liquid or the cell suspension flows in use, the hollow body in particular being a pipe or a tube.

7. The hollow body according to claim 6, being an actuatable part of an apparatus for applying an external action on the bodily liquid or the cell suspension.

8. A method for hydrophilically coating a hollow body for temporarily accommodating therein a bodily liquid at its inner surface, the method comprising:
- pre-coating the inner surface using a reactive gas plasma (PECVD); and
- follow-up-coating the pre-coated surface with a reactive gas component without plasma action (CVD).

9. The method according to claim 8, wherein the pre-coating has a duration of less than 10 minutes or less than 1 minute.

10. The method according to claim 8 or 9, wherein the reactive gas component is acrylic acid or the anhydride thereof.

11. The method according to one of claims 8 to 10, wherein the surface material of the hollow body is a synthetic resin.

12. Use of acrylic acid or acrylic acid anhydride for hydrophilizing inner surfaces of a hollow body for temporarily accommodating therein a bodily liquid, the use comprising:
- pre-coating the surfaces using a high frequency plasma generated from a gas mixture composed of an inert gas and a first gas formed of biocompatible, polymerizable, carboxy group-containing monomers, and then
- follow-up coating the pre-coated surfaces of the body,
wherein the pre-coating takes less than 10 minutes, and the follow-up coating is made using a second gas substantially consisting of the acrylic acid or acrylic anhydride monomers.

13. The use of claim 12, wherein there is no preceding plasma-activation step in the absence of the first gas or with less than 10 % of the gas formed of the monomers.

14. The use of claim 12, wherein there is a preceding plasma-activation step, using an inert gas plasma in the absence of the first gas or with less than 10 % of the gas formed of the monomers.

15. The use of one of claims 12 to 14, wherein the body is hollow, and the surfaces so coated are inner surfaces of the body, the use optionally further comprising temporarily accommodating in the hollow body a bodily liquid, in particular blood, or a cell suspension, wherein the temporarily accommodating optionally includes filling the bodily liquid or cell suspension into the hollow body, storing it, and later discharging it from the hollow body, such as in opposite direction to the filling, or includes steadily flowing the bodily liquid or cell suspension through the hollow body, the use further optionally comprising applying an external action such as pressure, heat, or radiation on the bodily liquid or the cell suspension.

## Patentansprüche

1. Hohlkörper zur temporären Aufnahme einer Körperflüssigkeit, insbesondere Blut, oder von Zellsuspensionen, wobei der Hohlkörper eine hydrophilierende Zweischritt-PECVD/CVD-Beschichtung an seiner inneren Oberfläche aufweist, die durch das Verfahren nach einem der Ansprüche 8 bis 11 erhältlich ist.

2. Hohlkörper nach Anspruch 1, wobei ein Wasserkontaktwinkel der beschichteten inneren Oberfläche weniger als 2°, insbesondere weniger als 0,5°, beträgt.

3. Hohlkörper nach einem der Ansprüche 1 bis 2, welcher eine polymere Beschichtung aus Acrylsäure-Monomeren aufweist.

4. Hohlkörper nach einem der Ansprüche 1 bis 3, wobei ein Oberflächenmaterial des Hohlkörpers ein synthetisches Harz ist.

5. Hohlkörper nach einem der Ansprüche 1 bis 4, welcher genau eine Öffnung aufweist, wobei der Hohlkörper insbesondere ein Beutel oder eine Flasche ist.

6. Hohlkörper nach einem der Ansprüche 1 bis 4, welcher zwei Öffnungen aufweist, zwischen denen die Körperflüssigkeit oder die Zellsuspension während der Benutzung strömt, wobei der Hohlkörper insbesondere ein Rohr oder ein Schlauch ist.

7. Hohlkörper nach Anspruch 6, welcher ein aktivierbarer Teil einer Vorrichtung zur Beaufschlagung der Körperflüssigkeit oder der Zellsuspension mit einer äußeren Einwirkung ist.

8. Verfahren zur hydrophilierenden Beschichtung eines Hohlkörpers zur temporären Aufnahme einer Körperflüssigkeit an seiner inneren Oberfläche, wobei das Verfahren aufweist:
- Vorbeschichten der inneren Oberfläche mit Hilfe von einem reaktiven Gasplasma (PECVD), und
- Nachbeschichten der vorbeschichteten Oberfläche mit einer reaktiven Gaskomponente ohne Plasmaeinwirkung (CVD).

9. Verfahren nach Anspruch 8, wobei das Vorbeschichten über eine Dauer von weniger als 10 Minuten oder weniger als 1 Minute erfolgt.

10. Verfahren nach Anspruch 8 oder 9, wobei die reaktive Gaskomponente Acrylsäure oder deren Anhydrid ist.

11. Verfahren nach einem der Ansprüche 8 bis 10, wobei das Oberflächenmaterial des Hohlkörpers ein synthetisches Harz ist.

12. Verwendung von Acrylsäure oder Acrylsäureanhydrid zur Hydrophilierung von inneren Oberflächen eines Hohlkörpers zur temporären Aufnahme einer Körperflüssigkeit, wobei die Verwendung umfasst:
- Vorbeschichten der Oberflächen unter Verwendung eines Hochfrequenzplasmas, welches aus einem Gasgemisch erzeugt wird, das aus einem inerten Gas zusammengesetzt ist und einem ersten Gas, welches aus biokompatiblen, polymerisierbaren, Carboxygruppen aufweisenden Monomeren gebildet ist, und dann
- Nachbeschichten der vorbeschichteten Oberflächen des Körpers,
wobei das Vorbeschichten weniger als 10 Minuten dauert, und das Nachbeschichten ausgeführt wird, indem ein zweites Gas verwendet wird, welches im Wesentlichen aus der Acrylsäure oder den Acrylanhydridmonomeren besteht.

13. Verwendung nach Anspruch 12, wobei es keinen vorangehenden Plasmaaktivierungsschritt in Abwesenheit des ersten Gases oder mit weniger als 10 % des aus den Monomeren gebildeten Gases gibt.

14. Verwendung nach Anspruch 12, wobei es einen vorangehenden Plasmaaktivierungsschritt gibt, bei dem ein Plasma eines inerten Gases in Abwesenheit des ersten Gases oder mit weniger als 10 % des aus den Monomeren gebildeten Gases verwendet wird.

15. Verwendung nach einem der Ansprüche 12 bis 14, wobei der Körper hohl ist und die so beschichteten Oberflächen innere Oberflächen des Körpers sind, wobei die Verwendung wahlweise weiterhin die temporäre Aufnahme einer Körperflüssigkeit, insbesondere Blut, oder von einer Zellsuspension, in dem Hohlkörper umfasst, wobei die temporäre Aufnahme wahlweise das Einfüllen der Körperflüssigkeit oder Zellsuspension in den Hohlkörper, das Lagern derselben und später das Freisetzen derselben aus dem Hohlkörper z.B. in umgekehrter Richtung zum Einfüllen umfasst, oder das kontinuierliche Strömen der Körperflüssigkeit oder der Zellsuspension durch den Hohlkörper umfasst, wobei die Verwendung wahlweise weiterhin das Beaufschlagen der Körperflüssigkeit oder der Zellsuspension mit einer äußeren Einwirkung, wie Druck, Wärme oder Strahlung, umfasst.

## Revendications

1. Corps creux destiné à loger temporairement un fluide corporel, en particulier du sang, ou des suspensions de cellules, le corps creux étant muni d'un revêtement hydrophilisant à deux étapes PECVD/CVD sur sa surface intérieure qui peut être obtenu par le procédé selon l'une quelconque des revendications 8 à 11.

2. Corps creux selon la revendication 1, dans lequel l'angle de contact avec l'eau de la surface intérieure revêtue est inférieur à 2°, en particulier inférieur à 0,5°.

3. Corps creux selon l'une quelconque des revendications 1 à 2, muni d'un revêtement polymère constitué de monomères d'acide acrylique.

4. Corps creux selon l'une quelconque des revendications 1 à 3, dans lequel un matériau de surface du corps creux est une résine synthétique.

5. Corps creux selon l'une quelconque des revendications 1 à 4, muni d'exactement une ouverture, le corps creux étant en particulier une poche ou une bouteille.

6. Corps creux selon l'une quelconque des revendications 1 à 4, muni de deux ouvertures, entre lesquelles le fluide corporel ou la suspension de cellules s'écoule à l'usage, le corps creux étant en particulier un tuyau ou un tube.

7. Corps creux selon la revendication 6, étant une partie actionnable d'un appareil destiné à appliquer une action externe sur le fluide corporel ou la suspension de cellules.

8. Procédé de revêtement hydrophile d'un corps creux destiné à loger temporairement un fluide corporel sur sa surface intérieure, le procédé comprenant :
- le pré-revêtement de la surface intérieure en utilisant un plasma gazeux réactif (PECVD) ; et
- le revêtement complémentaire de la surface pré-revêtue avec un composant gazeux réactif sans action de plasma (CVD).

9. Procédé selon la revendication 8, dans lequel le pré-revêtement a une durée de moins de 10 minutes ou de moins de 1 minute.

10. Procédé selon la revendication 8 ou 9, dans lequel le composant gazeux réactif est l'acide acrylique ou son anhydride.

11. Procédé selon l'une quelconque des revendications 8 à 10, dans lequel le matériau de surface du corps creux est une résine synthétique.

12. Utilisation d'acide acrylique ou d'anhydride d'acide acrylique pour hydrophiliser des surfaces intérieures d'un corps creux destiné à loger temporairement un fluide corporel, l'utilisation comprenant :
- le pré-revêtement des surfaces en utilisant un plasma haute fréquence généré à partir d'un mélange gazeux composé d'un gaz inerte et d'un premier gaz formé par des monomères biocompatibles polymérisables contenant un groupe carboxy, puis
- le revêtement complémentaire des surfaces pré-revêtues du corps,
dans laquelle le pré-revêtement prend moins de 10 minutes, et le revêtement complémentaire est réalisé en utilisant un second gaz essentiellement constitué par les monomères d'acide acrylique ou d'anhydride acrylique.

13. Utilisation selon la revendication 12, dans laquelle il n'y a pas d'étape d'activation plasma préalable en l'absence du premier gaz ou avec moins de 10 % du gaz formé par les monomères.

14. Utilisation selon la revendication 12, dans laquelle il y a une étape d'activation plasma préalable, utilisant un plasma gazeux inerte en l'absence du premier gaz ou avec moins de 10 % du gaz formé par les monomères.

15. Utilisation selon l'une quelconque des revendications 12 à 14, dans laquelle le corps est creux, et les surfaces ainsi revêtues sont des surfaces intérieures du corps, l'utilisation comprenant de manière optionnelle le logement temporaire dans le corps creux d'un fluide corporel, en particulier de sang, ou d'une suspension de cellules, dans laquelle le logement temporaire comprend de manière optionnelle le remplissage du corps creux avec le fluide corporel ou la suspension de cellules, le stockage de celui-ci ou de celle-ci et son déchargement ultérieur du corps creux, tel que dans la direction opposée au remplissage, ou il comprend l'écoulement continu du fluide corporel ou de la suspension de cellules dans le corps creux, l'utilisation comprenant de manière optionnelle en outre l'application d'une action extérieure telle qu'une pression, une chaleur ou un rayonnement sur le fluide corporel ou la suspension de cellules.
